(19) Europäisches Patentamt
European Patent Office
Office européen des brevets

(11) Veröffentlichungsnummer: **0 156 215**
**A1**

(12) **EUROPÄISCHE PATENTANMELDUNG**

(21) Anmeldenummer: **85102563.5**

(22) Anmeldetag: **07.03.85**

(51) Int. Cl.⁴: **C 07 C 121/413**

(30) Priorität: **16.03.84 DE 3409716**

(43) Veröffentlichungstag der Anmeldung:
**02.10.85 Patentblatt 85/40**

(84) Benannte Vertragsstaaten:
**BE DE FR GB IT NL**

(71) Anmelder: **BAYER AG**
**Konzernverwaltung RP Patentabteilung**
**D-5090 Leverkusen 1 Bayerwerk(DE)**

(72) Erfinder: **Waniczek, Helmut, Dr.**
**Wolfskaul 2**
**D-5000 Koeln 80(DE)**

(72) Erfinder: **Bartl, Herbert, Dr.**
**Eichendorffweg 10**
**D-5068 Odenthal(DE)**

(54) **Verfahren zur Herstellung von Alpha-Cyanacrylsäureestern.**

(57) Die Erfindung betrifft ein neues Verfahren zur Herstellung von α-Cyanacrylsäureestern.

EP 0 156 215 A1

BAYER AKTIENGESELLSCHAFT 5090 Leverkusen, Bayerwerk

Konzernverwaltung RP
Patentabteilung Eck-klu/c

Verfahren zur Herstellung von α-Cyanacrylsäureestern

Die Erfindung betrifft ein neues Verfahren zur Herstellung von α-Cyanacrylsäureestern.

α-Cyanacrylsäureester durch thermisch-katalytische Depolymerisation von Polycyanacrylaten herzustellen ist bekannt (z.B. DE-AS 2 027 502, US-PS 2 721 858, DE-AS 2 104 518). Bei diesen Verfahren wird Polycyanacrylat gemeinsam mit Anionenfängern wie $P_2O_5$ oder Polyphophorsäure und Radikalfängern wie Hydrochinon in Gegenwart eines flüchtigen Anionenfängers wie $SO_2$ im Vakuum erhitzt. Oberhalb 150°C destillieren die Depolymerisationsprodukte ab. Diese Depolymerisationsdämpfe werden normalerweise kondensiert und gesammelt. Um eine hohe Ausbeute an monomeren Cyanacrylat zu erzielen, muß dabei die Temperatur des Reaktionsgemisches auf 200°C bis 290°C erhöht werden.

Da α-Cyanacrylate sehr leicht zur Polymerisation neigen, können bei dieser Art der Herstellung die Depolymerisationsdämpfe beim Kondensieren polymerisieren und so z.B. die Apparatur zusetzen, wie in der DE-OS 1 568 479 beschrieben. Diese Polymerisation kann zwar durch einen

weiteren Zusatz von sauren Verbindungen wie Phosphorsäure oder $SO_2$ wieder unterdrückt werden, jedoch wird die Lagerstabilität der so hergestellten Cyanacrylate außerordentlich verschlechtert, wenn sich bereits Polymerisat gebildet hat. Weiterhin wird durch die Polymerisation die Ausbeute an monomerem Cyanacrylat vermindert. Außerdem können bei der Depolymerisation Nebenprodukte entstehen, die die Polymerisation fördern können. Gelangen diese Spaltnebenprodukte in das kondensierte $\alpha$-Cyanacrylat, können sie dort eine Polymerisation begünstigen. So hergestelltes $\alpha$-Cyanacrylat ist schlecht lagerfähig. Wegen der Bildung dieser Spaltnebenprodukte sind daher besondere Reinigungsmaßnahmen nötig. Ohne diese Maßnahmen ist die Ausbeute an Cyanacrylat sehr gering (z.B. DE-AS 2 027 502).

Es wurde nun gefunden diese Nachteile zu beseitigen, indem die bei der Depolymerisation entstehenden heißen Dämpfe durch eine Zone mit einer Temperatur geführt werden, bei der hochsiedende Anteile kondensieren und abgetrennt werden können, aber das Cyanacrylat noch dampfförmig bleiben und getrennt kondensiert werden kann.

Gegenstand der Erfindung ist ein Verfahren zur Herstellung von $\alpha$-Cyanacrylsäureestern der Formel I

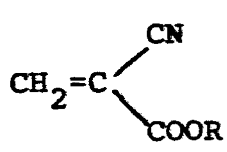

(I),

in welcher

R ein gesättigter oder ungesättigter Alkyl- oder Cycloalkylrest mit 1 bis 8 C-Atomen ist,

durch Depolymerisation von Polycyanacrylaten der Formel II

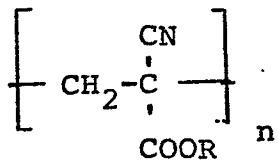

(II),

in welcher

R die bei Formel (I) angegebene Bedeutung hat und

n für eine ganze Zahl von 3 bis 80 steht,

in Gegenwart von Radikalinhibitoren, Anionenfängern und gegebenenfalls Wärmeträgern bei einer Temperatur von 150 bis 320°C und einem Druck von 0.1 bis 50 mbar, dadurch gekennzeichnet, daß die entstehenden Spaltdämpfe durch eine Zone mit einer Temperatur von 120°C bis 200°C bevorzugt von 150°C bis 190°C geleitet werden und anschließend in einer Zone mit einer Temperatur von -30°C bis 50°C, bevorzugt von -15°C bis 30°C kondensiert werden.

Die Depolymerisation wird bei einem Druck von 0,1 bis 150 mbar, vorzugsweise 0,5 bis 50 mbar, besonders bevorzugt 0,5 bis 20 mbar ausgeführt.

Als Radikalinhibitoren können übliche Radikalfänger eingesetzt werden, z.B. Phenole wie Hydrochinon, p-Methoxyphenol, di-tert.-butylphenol und tert.-Butylbrenzkatechin, vorzugsweise Hydrochinon.

Unter Anionenfängern sind beispielsweise Phosphorverbindungen wie Phosphorpentoxid, Polyphosphorsäuren, Antimonverbindungen wie Antimonpentoxid, organische Säuren wie Pikrinsäure, Maleinsäureanhydrid, anorganische Verbindungen wie Eisen(III)-chlorid, $SO_2$ oder Fluorwasserstoff, bevorzugt substituierte oder unsubstituierte Aryl- oder Alkylsulfonsäuren wie Methansulfonsäure, Trifluormethansulfonsäure oder p-Toluolsulfonsäure oder Alkylsulfonsäureanhydride wie Methansulfonsäureanhydrid, Nonafluorbutansulfonsäureanhydrid oder p-Toluolsulfonsäureanhydrid genannt.

Als Wärmeträger können hochsiedende organische Lösungsmittel wie Trikresylphosphat, Triphenylphosphat, Ethylenglykoldimethylether oder Triethylenglykoldimethylether eingesetzt werden.

Das erfindungsgemäße Verfahren kann wie folgt durchgeführt werden:
Polycyanacrylat, ein Radikalinhibitor, ein Anionenfänger und gegebenenfalls ein Wärmeträger werden in einen Rührreaktor (I) vorgelegt. Von dem Reaktordeckel führt ein kurzes, gebogenes Rohr in ein Rührgefäß (II), welches mit einem absteigenden Kühler oder einem Kolonnenkopf versehen ist, an dem eine Vorlage mit einem Vakuumvorstoß montiert ist. Das Rührgefäß (II) wir z.B. mit einem Ölbad bei einer Temperatur von 120°C bis 200°C gehalten. Die Apparatur wird evakuiert und der Rührreaktor auf Reaktionstemperatur gebracht. Während der

Depolymerisationsreaktion destillieren bei diesen Temperatur/Druckverhältnissen flüchtige Bestandteile in das Rührgefäß II. Dieses Rührgefäß (II) wird bei einer Temperatur von mehr als 120°C und weniger als 200°C, vorzugsweise 150 bis 190°C gehalten, so daß hochsiedende Bestandteile des eingeleiteten Dampfes auskondensieren können. Der so gereinigte Dampf enthält das monomere Cyanacrylat und kann am Kühler oder Kolonnenkopf auskondensiert und in einer Vorlage gesammelt werden.

Eine bevorzugte Durchführung des erfindungsgemäßen Verfahrens besteht darin, daß die bei der Spaltung von Polycyanacrylat entstehenden Spaltdämpfe kontinuierlich in ein gerührtes Gefäß geleitet werden, welches durch Beheizung bei einer Temperatur von mehr als 120°C, aber weniger als 200°C, bevorzugt 150°C bis 190°C gehalten wird, und daß die so gereinigten Dämpfe getrennt kontinuierlich kondensiert und abgetrennt werden können.

Bei der Durchführung des erfindungsgemäßen Verfahrens ist es möglich, in dem Sammelgefäß für das gereinigte Cyanacrylatkondensat einen zusätzlichen Stabilisator zuzugeben.

Die nach dem erfindungsgemäßen Verfahren erhaltenen $\alpha$-Cyanacrylate sind bei Raumtemperatur mehr als 10 Wochen lagerstabil. Sie können ohne weitere Destillation als Klebstoffe weiterverarbeitet werden. Bei einer Reinigung durch Destillation liefern die erfindungsgemäß hergestellten Cyanacrylate höhere Ausbeuten durch die verbesserte Lagerstabilität.

Besonders vorteilhaft kann das erfindungsgemäße Verfahren zur Herstellung von Cyanacrylsäuremethylester, -pentylester, -hexylester, -octylester oder -benzylester, die unsubstituiert oder mit z.B. Halogenen substituiert sein können, eingesetzt werden.

Der Vorteil der vorliegenden Erfindung gegenüber dem Stand der Technik liegt darin, daß es gelingt, Cyanacrylate mit einer weit höheren Verfahrenssicherheit herzustellen. Polymerisation der Monomeren kann verhindert werden, die erfindungsgemäß hergestellten Cyanacrylate weisen eine verbesserte Lagerstabilität auf und können z.B. durch Destillation besser weiterverarbeitet werden.

Die nach dem erfindungsgemäßen Verfahren hergestellten $\alpha$-Cyanacrylate eignen sich bereits ohne weitere Destillation zur Verwendung als lagerfähige schnell abbindende Einkomponenten-Klebstoffe. Ihre Abbindegeschwindigkeit kann durch eine weitere destillative Reinigung noch erhöht werden.

Zur Verleihung bestimmter Eigenschaften können diese Klebstoffe noch weitere Substanzen wie Stabilisatoren, Füllstoffe, Weichmacher oder Polymere zur Einstellung einer bestimmten Viskosität enthalten. Derartige Zusätze sind bekannt.

Beispiel 1

Eine Apparatur bestehend aus einem 4-ltr.-Dreihalskolben (1), der mit einem Rührer, einem Gaseinleitrohr und einem etwa 15 cm langen, um 180°C gebogenen Rohr (5), welches in einen Schliff eines 1 ltr.-Dreihalskolbens (2) führt, wird mit 2300 Polycyanacrylsäuremethylester, 100 g $P_2O_5$, 100 g Hydrochinon und 800 g Trikresylphosphat gefüllt. Der 1 ltr. Dreihalskolben ist mit einem Rührer und einer Destillationsbrücke (3) mit einem 3 ltr. Vorlagekolben (4) ausgestattet und wird mit einem Ölbad bei einer Temperatur von 170°C bis 180°C gehalten.

Nun wird die Apparatur auf ca. 1 mbar evakuiert, durch das Gaseinleitrohr ein schwacher $SO_2$-Strom eingeleitet, und unter Rühren die Temperatur des 4 ltr.-Kolbens gesteigert. Bei einer Innentemperatur von 155°C beginnt der Spaltdampf aus dem 4 ltr.-Kolben durch das Verbindungsrohr in den 1 ltr.-Kolben zu strömen. Dort wird ein hellbraunes Öl abgeschieden, während das monomere Cyanacrylat im Kühler kondensiert und in der Vorlage gesammelt wird.

Die Temperatur im 4 ltr.-Kolben wird im Verlauf der Reaktion bis auf 220°C gesteigert. Nach Beendigung der Reaktion sind in dem Vorlagekolben 1965 g fast farbloser $\alpha$-Cyanacrylsäuremethylester, während in dem 1 ltr.-Kolben 650 g eines gelben Öls sind.

Nach diesem Versuch ist in der Apparatur noch keine Polymerisatbildung festzustellen.

Nach einer Lagerung von 3 Wochen bei 23°C wurde das so hergestellte Cyanacrylat im Vakuum destilliert, wobei 1867 g reiner α-Cyanacrylsäuremethylester erhalten wurden. Dies entspricht einer Ausbeute von 81 %.

Beispiel 2

Der Versuch aus Beispiel 1 wird mit Polycyanacrylsäureethylester wiederholt. Es liegen danach 1890 g α-Cyanacrylsäureethylester in dem Vorlagekolben vor, während der 1 ltr.-Kolben 702 g eines gelben Öles enthält.

Der Apparatur ist keine Polymerbildung anzusehen.

Nach 3-wöchiger Lagerung bei 23°C wurden durch Vakuumdestillation 1795 g α-Cyanacrylsäureethylester erhalten, was einer Ausbeute von 78 % entspricht.

Klebeversuch:
Mit den Cyanacrylaten aus Beispiel 1 und 2 wurde vor und nach der Destillation ein Klebeversuch durchgeführt, indem ein Tropfen des Klebstoffes auf eine Platte aus Polymethylmethacrylat (PMMA) aufgetragen und eine zweite Platte aus PMMA auf den Tropfen gedrückt wurde. Die beiden Platten waren nach wenigen Sekunden fest miteinander verbunden.

Beispiel 3

Der Versuch aus Beispiel 1 wurde wiederholt. Danach wurde die Apparatur mit trockener Luft belüftet und die

Gefäße (1), (2) und (4) durch die Bodenablaßventile entleert. Ohne die Versuchsapparatur zu reinigen wurde der Versuch mehrmals wiederholt, ohne daß Polymerisatbildung zu beobachten war. Die Ausbeute lag nach einer Vakuumdestillation immer über 80 %.

Vergleichsbeispiel 1

Der Versuch aus Beispiel 1 wurde wiederholt, wobei aber die Temperatur des Kolbens (2) bei 200°C gehalten wurde. Während des Versuches bildete sich im Kühler (3) Polymerisat. Das Zusetzen des Kühlers konnte nur durch eine hohe $SO_2$-Dosierung verhindert werden.

Vergleichsbeispiel 2 gemäß der DOS 1 568 479

Zu 396 Teilen (4 Molen) Methylcyanoacetat, 350 Teilen Benzol, 1 Teil Piperidin und 1 Teil 50 %iger Natriumhydroxydlösung, die sich in einem 1-Liter-Kolben befanden, der mit einem Rührwerk, einem Rückflußkühler und einer Wasserfalle versehen war, wurden 100 Teile (3,33 Mol; molares Verhältnis 1:1,2) Paraformaldehyd in 4 Portionen zugegeben. Zur kontinuierlichen Entfernung des gebildeten Wassers wurde auf Rückflußtemperatur erhitzt. Nachdem das bei der Reaktion gebildete Wasser praktisch vollständig entfernt worden war, wurde die heiße Lösung dekantiert, wobei die festen Katalysatorteilchen zurückblieben. Bei kräftigem Rühren wurden anschließend 20 Teile Polyphosphorsäure hinzugegeben. Nach 10-minütigem Rühren wurde soviel Phosphorpentoxyd zugegeben, daß die Polyphosphorsäure und Phosphate an den Behälterwänden kleben blieben. Anschließend wurde die Lösung dekantiert, das Benzol wurde abgezogen und der Rückstand durch Erhitzen auf 170 bis 185°C unter Vakuum in einem schwachen $SO_2$-Strom pyrolisiert. Das Destillat wurde in einer gekühlten Vorlage, die 5 Teile Phosphorpentoxyd und 1 Teil Hydrochinon enthielt, aufgefangen.

Wenn in der Destillationsbrücke eine Polymerisatbildung zu beobachten war, wurde der $SO_2$-Strom jeweils verstärkt. Das Destillat war nach 2 Tagen zäh und nach 1 Woche zu einer harten, spröden Masse polymerisiert.

Der Versuch wurde wiederholt und das rohe Cyanacrylat sofort nach der Herstellung im $SO_2$-Strom und im Vakuum destilliert. Dabei wurden 219 g reiner Cyanacrylsäuremethylester isoliert, was einer Ausbeute von 59 % entspricht.

Bei mehreren Wiederholungen des Versuches konnte die Polymerisatbildung im Kühler auch durch hohe $SO_2$-Dosierung nicht gestoppt werden. Es wurde kein monomeres Cyanacrylat isoliert.

Beispiel 4

Ein Entgasungsextruder wird kontinuierlich mit 6 kg/h eines Gemisches aus 90 Gew.-% Polycyanacrylsäuremethylester, 5 Gew.-% $P_2O_5$ und 5 Gew.-% Hydrochinon beschickt. Kurz nach der Einzugsöffnung des Extruders werden mit einer Pumpe 300 ml/h einer Lösung von 10 Gew.-% $SO_2$

in 90 Gew.-% Trikresylphosphat in das Reaktionsgemisch gepumpt.

Das Entgasungsgehäuse des Extruders wird auf 300°C beheizt. Von dem Entgasungsturm führt ein Rohr an den Deckel eines gerührten 5 ltr.-Planschliffkolbens, der von außen auf 185°C beheizt wird, und der an einer weiteren Öffnung im Deckel einen Kolonnenkopf mit einem Vorlagekolben trägt, an dem Vakuum von 1 mbar angelegt wird.

Durch die Düse des Extruders wird der Depolymerisationsrückstand ausgetragen, während in dem 5 ltr.-Planschliffkolben pro Stunde 540 g eines gelben Öles aufgefangen werden. In der Vorlage werden pro Stunde 4100 g α-Cyanacrylsäuremethylester gesammelt.

Vergleichsbeispiel 3

Der Entgasungsextruder aus Beispiel 4 wird mit den gleichen Mengen der Substanzen aus Beispiel 4 beschickt.

Vom Entgasungsturm des Extruders führt nun direkt ein Rohr zu einem absteigenden Kühler, der seinerseits mit einer Vorlage und einem Vakuumvorstoß versehen ist. Unter den gleichen Versuchsbedingungen wie in Beispiel 4 war der Kühler 20 Minuten nach dem Anfahren der Versuchsanlage zupolymerisiert. Das bis dahin in der Vorlage gesammelte α-Cyanacrylat (810 g) war ebenfalls polymerisiert.

## Patentanspruch

1) Verfahren zur Herstellung von -Cyanacrylsäureestern der Formel I

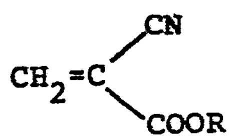

(I),

in welcher

R ein gesättigter oder ungesättigter Alkyl- oder Cycloalkylrest mit 1 bis 8 C-Atomen ist,

durch Depolymerisation von Polycyanacrylaten der Formel II

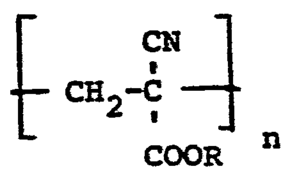

(II),

in welcher R die bei Formel (I) angegebene Bedeutung hat und

n für eine ganze Zahl von 3 bis 80 steht,

in Gegenwart von Radikalinhibitoren, Anionenfängern und gegebenenfalls Wärmeträgern bei einer Temperatur 150°C bis 320 °C und einem Druck von 0.1 bis

150 mbar, dadurch gekennzeichnet, daß die entstehenden Spaltdämpfe durch eine Zone mit einer Temperatur von 120°C bis 200°C und anschließend in eine Zone mit einer Temperatur von -30°C bis 50°C geleitet werden.

Europäisches Patentamt

# EUROPÄISCHER RECHERCHENBERICHT

0156215
Nummer der Anmeldung

EP 85 10 2563

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| | | | C 07 C 121/413 |
| X,D | DE-A-2 104 518 (SCHERING AG) * Seiten 7,11 * | 1 | |
| X | FR-A-2 093 870 (SCHERING AG) * Seite 6, Beispiel 1 * & DE - A - 2 027 502 (Kat. D) | 1 | |
| A | DE-A-2 738 285 (WINKOVIC) * Seite 5, Zeilen 4-8; Seite 6, letzter Absatz * | 1 | |
| A | US-A-2 763 677 (JEREMIAS) * Spalte 5, Zeilen 5-15 * | 1 | |
| A | US-A-2 784 215 (JOYNER) * Spalte 5, Zeilen 57-60 * | 1 | RECHERCHIERTE SACHGEBIETE (Int. Cl.4) |
| A | FR-A-1 535 001 (AMERICAN CYANAMID CO.) * Seite 4, Beispiel 7 * | 1 | C 07 C 121/00 |
| A,D | US-A-2 721 858 (JOYNER u.a.) * Spalte 5, Zeilen 36-38 * | 1 | |

Der vorliegende Recherchenbericht wurde für alle Patentansprüche erstellt.

| Recherchenort | Abschlußdatum der Recherche | Prüfer |
|---|---|---|
| DEN HAAG | 28-05-1985 | THEUNS H.G. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument